# EUROPEAN PATENT SPECIFICATION

(11) **EP 1 790 376 B1**
(45) Date of publication and mention of the grant of the patent: **14.04.2010**
(21) Application number: 06022929.1
(22) Date of filing: 03.11.2006
(51) Int. Cl.: A61M 39/12, A61M 39/10, A61J 15/00

(54) **Single lumen adapter for automatic valve**
Einlumiger Adapter für ein automatisches Ventil
Adapteur à lumière unique pour valve automatique

(30) Priority: 23.11.2005 US 286094
(43) Date of publication of application: 30.05.2007
(73) Proprietor: Covidien AG, 8212 Neuhausen am Rheinfall (CH)
(72) Inventor: Hanlon, James G., Manchester, MO 63021 (US); Swisher, David Rork, St.Charles, MO 63301 (US); Meier, Kevin C, Afton, MO 63123 (US); Fournie, Glenn G, Smithton, IL 62285 (US)
(74) Representative: Tomlinson, Edward James

(56) References cited:
- WO-A2-2004/039286
- GB-A- 1 262 146
- US-A- 4 617 012
- US-A- 5 088 486
- US-A- 5 217 432
- US-A- 5 256 160
- US-A- 5 322 073
- US-A1- 2003 153 897
- US-A1- 2003 236 500
- US-A1- 2004 186 457
- US-A1- 2004 201 216
- US-A1- 2005 171 468
- US-A1- 2005 245 899
- US-B1- 6 332 467

## Description

### Technical Field

The present disclosure generally relates to medical administration of fluids with a subject, and more particularly, to a single lumen adapter for a valve system, having multiple ports, which is manipulated to establish fluid communication with a passageway of a nasogastric tube.

### Description of the Related Art

Medical systems inserted with a body of a subject for the administration of fluids with the subject, such as, for example, nasogastric tubing are known in the art. Nasogastric tubing is typically employed in hospitals, nursing homes, care facilities, etc. to remove fluids from the body of the subject, such as, for aspirating fluids from a gastrointestinal tract (GI tract) of the subject or to introduce nutrients, supplements, medicines, etc. to the subject. In this regard, published applications WO 2004/039286 and WO 2005/102436 are referred to.

In one application, nasogastric tubing aspirates fluid and air to decompress the contents of the subject's stomach to avoid damaging the inner wall, e.g., the gastric mucosa. Nasogastric tubing may also facilitate removal of accumulated fluids, blood, etc. from the GI tract due to disease, intestinal obstruction, bleeding ulcers and paralytic ulcers to prevent progressive distension of the GI tract. Progressive distension of the GI tract can lead to shock, visceral injury and vomiting. Vomit may be aspirated into the respiratory tract and cause asphyxia and pneumonia.

Nasogastric tubes are employed with subjects undergoing abdominal surgery to keep the stomach vacant of fluid and postoperatively to prevent complications, such as, decreased gastrointestinal function. Such nasogastric tubing prevents pooling of liquids in the GI tract to facilitate postoperative recovery of digestive function. Nasogastric tubing can also be employed to protect gastric suture lines, preventing and treating paralytic ileus, treating drug overdoses, lavage, as well as other conditions that affect the GI tract.

In conventional use, a flexible plastic nasogastric tube is employed. The nasogastric tube defines a passageway that extends from a proximal end to a distal end. A practitioner introduces the distal end of the nasogastric tube through a nasal canal of a subject via one of the nostrils. The distal end is passed through the pharynx and down the esophagus into the GI tract. The distal end can be passed into the duodenum, stomach, etc. depending on the particular application such as, for example, aspirating fluids, introduction for medication, feeding, etc. Several openings are formed in the distal end that permit passage of gastric fluids, nutrients, medication, etc.

To prevent blockage of the openings in the distal end, a dual lumen nasogastric tube is generally used. The dual lumen nasogastric tube includes a suction/irrigation lumen and a separate vent lumen. The suction/irrigation lumen is connected to a suction source providing either intermittent or continuous suction to facilitate suction drainage and irrigation. The vent lumen communicates with the suction/ irrigation lumen adjacent the distal end of the nasogastric tubing to permit atmospheric air to be drawn through the vent lumen into the suction lumen. The flow of atmospheric air moderates the amount of suction and flow during aspiration. Nutrients or medication introduced is passed down the suction lumen and the vent lumen is clamped or plugged. Air pressure is applied thereafter to clear the vent lumen.

The proximal end of the nasogastric tube exits the nostril and communicates with a suction source. The proximal end may be connected to the suction source, a feeding pump, etc. through a connector that may communicate with a collection vessel. In a fluid aspirating application, stomach fluids are drawn through the openings in the distal end, through the passageway and into the collection vessel, as facilitated by the suction source. In a fluid introduction application, nutrients, medication, etc. are injected into the passageway and forced through the openings in the distal end and into for example, the duodenum.

The connector is connected to a second tube that is connected to the suction source, or alternatively, to a feeding pump. Frequently, the nasogastric tubing must be alternated to a source for suction, feeding or introduction of an injection. To alternate the nasogastric tubing application, the second tube is removed from the connector or the connector is removed from the proximal end of the nasogastric tubing and the desired connection is made. These known devices and methods suffer from many drawbacks. Typically, the practitioner is spattered with vomit or other fluid during disconnection of the tubing and connector.

This procedure may also require clamping of the tubing. This is disadvantageously cumbersome, unclean and does not adequately prevent leakage of GI tract fluids. Leaking and splattering intestinal fluids can cause contamination of wounds, tubing and catheters. The intestinal fluids may contain infectious material that poses serious health risks to the practitioner.

Another drawback of these devices and methods is the labor intensive burden of cleaning the leaking and splattering intestinal fluids. Patient discomfort and complication may also result. This consumes a great deal of practitioner time and adds to the cost of healthcare.

Therefore, it would be desirable to overcome the disadvantages and drawbacks of the prior art with a valve system, having multiple ports, which is manipulated to establish fluid communication with a passageway of a nasogastric tube to avoid leakage of intestinal fluids and minimize disease propagation. It would be desirable if such a valve system included a rotatable valve member that is manipulated to facilitate connection of the passageway of the nasogastric tube with alternate sources to achieve the principles of the present disclosure. It would be highly desirable if the valve system is connected to a second passageway of the nasogastric tube. It is contemplated that the valve system and its constituent parts are easily and efficiently manufactured and assembled.

### SUMMARY

A fluid adapter is provided for connection between a valve having a plurality of ports and a tube having a single lumen. The fluid adapter has a housing with a distal end and a proximal end and the proximal end has a first opening connecting to a first lumen being disposed through the housing. The proximal end has a second opening being separated by the first opening by a distance with the second opening connecting to a second lumen. The fluid adapter also has the distal end with the first lumen connecting to a single outlet. The second lumen terminates in the housing. The fluid adapter also has the single outlet of the housing connecting to the intermediate tube and the intermediate tube is connected to the tube to permit fluid to traverse through the tube and the valve. The housing further comprises a tab, said tab being releasably connected over a secondary port of the valve and wherein said tab is configured to occlude the secondary port to prevent the user form toggling the secondary port and said housing is configured to be releasably connected to the valve.

According to another aspect of the present disclosure, the fluid adapter has the intermediate tube connected to a member having a distal end with a neck portion configured to permit the tube having the single lumen to fasten over the neck portion.

According to another aspect of the present disclosure, the fluid adapter has the neck portion of the member having a plurality of flanges.

According to yet another aspect thereof, the fluid adapter has the plurality of flanges configured to form a barb-like configuration to retain the tube thereon in a sealed manner.

According to another aspect of the present disclosure, the fluid adapter has the plurality of flanges with at least one with a first diameter and at least another flange with a second diameter with the first diameter and the second diameter being differently sized diameters.

According to another aspect thereof, the fluid adapter has the plurality of flanges sized to facilitate retention of the tube.

According to another aspect of the present disclosure, the fluid adapter has the plurality of flanges with at least one with a first diameter and at least another flange with a second diameter. The first diameter and second diameter are differently sized diameters to facilitate retention of the tube.

According to another aspect thereof, the fluid adapter has the housing being generally cylindrical and sized to be manipulated by hand.

According to still another aspect of the present disclosure, the fluid adapter has the tube with a length. The length is suitable to rotate the valve being connected to the housing without disengaging the housing from the valve.

According to another aspect thereof, the fluid adapter has the first lumen being generally cylindrical.

According to another aspect of the present disclosure, the fluid adapter has a second lumen that is generally cylindrical and terminates at an end.

According to still another aspect thereof, the fluid adapter has the housing made from a flexible material.

According to still yet another aspect of the present disclosure, the fluid adapter has least one of the first lumen and the second lumen with a first size being relatively smaller than another of the first lumen and the second lumen to accommodate the plurality of ports of the valve.

According to another aspect thereof, the fluid adapter has at least one lumen terminating at an end to close at least one of the plurality of ports of the valve.

According to another aspect of the present disclosure, the fluid adapter has the intermediate tube connected to a flanged member with a plurality of flanges to permit the tube having the single lumen to fasten over the plurality of flanges.

According to still another aspect thereof, the fluid adapter has the intermediate tube being transparent and configured to permit a visual inspection thereof.

According to another aspect of the present disclosure there is provided a fluid adapter for connection between a valve and a tube. The fluid adapter has a housing. The housing has a distal end and a proximal end. The proximal end has a first opening connecting to a first lumen that is disposed through the housing. The proximal end has a second opening being separated by the first port by a distance. The second opening is a close ended or blind port and connects to a second lumen. The distal end has the first lumen connected to a single outlet with the outlet connecting to the tube having a single lumen. The housing is configured to be releasably connected to the valve.

According to another aspect thereof, the fluid adapter has the housing made from a thermoplastic.

According to another aspect of the present disclosure, the fluid adapter has the housing made from a material selected from the group consisting of a phthalate free polyvinyl chloride, a HDPE plastic, a PP plastic, and any combination thereof.

According to another aspect thereof, the fluid adapter has one opening with a compression fit, and wherein another opening has a relatively looser fit relative to the compression fit.

According to another aspect of the present disclosure, the fluid adapter has a single outlet with a neck portion with a plurality of flanges and the plurality of flanges form a barb like configuration to connect with the tube.

### BRIEF DESCRIPTION OF THE FIGURES

The objects and features of the present disclosure, which are believed to be novel, are set forth with the particularity in the appended claims. The present disclosure, both as to its organization and manner of operation, together with further objectives and advantages, may be best understood by reference to the following description, taken in connection with the accompanying drawings, which are described below.
FIG. 1 is a perspective view of one embodiment of a prior art valve system;
FIG. 2 is a perspective view of the valve system shown in FIG. 1, with parts separated;
FIG. 3 is a front view of a distal end of a valve of the valve system shown in FIG. 1;
FIG. 4 is a cutaway side view of a proximal end of the valve shown in FIG. 1;
FIG. 5 is a cutaway side view of an alternate embodiment of the proximal end shown in FIG. 4;
FIG. 6 is a side perspective view of a cap of the valve shown in FIG. 1;
FIG. 7 is a bottom perspective of the cap shown in FIG. 6;
FIG. 8 is a top perspective view of a part of the valve shown in FIG. 1;
FIG. 9 is a bottom perspective view of the part shown in FIG. 8;
FIG. 10 is a perspective view of a body of the valve shown in FIG. 1;
FIG. 11 is a perspective view of the valve system having the cap rotated for introduction;
FIG. 12 is a cutaway perspective view of the valve system shown in FIG. 11 having a cannula inserted therewith;
FIG. 13 is a perspective view of an alternate embodiment of the valve system, in accordance with the principles of the present disclosure;
FIG. 14 is a perspective view of the valve system shown in FIG. 13, with parts separated;
FIG. 15 is a top cross-sectional view of the valve system shown in FIG. 13;
FIG. 16 is a perspective view of an alternate embodiment of the valve system shown in FIG. 13;
FIG. 17 is a perspective view of the valve system shown in FIG. 13 including a nasogastric tube;
FIG. 18 is a perspective cutaway view of a portion of the nasogastric tube and an adapter shown in FIG. 17;
FIG. 19 is a perspective view of an alternate embodiment of the valve system shown in FIG. 13 with a housing section removed;
FIG. 20 is a perspective view of the valve system shown in FIG. 19, with parts separated;
FIG. 21 is a perspective view of an adapter according to the present disclosure having a tab for connection to the valve system shown in FIG. 17;
FIG. 22 is a rear view of the adapter of FIG. 21;
FIG. 23 is a front view of the adapter of FIG. 21;
FIG. 24 is a top view of the adapter of FIG. 21;
FIG. 25 is a bottom view of the adapter of FIG. 21;
FIG. 26 is a perspective view of the adapter of FIG. 21 being mated with the valve system of FIG. 17;
FIG. 27 is a cross sectional view of the adapter of FIG. 21;
FIG. 28 is a cross sectional view of the adapter being mated to the valve system of FIG. 26;
FIG. 29 is a perspective view of another embodiment of the adapter of FIG. 21;
FIG. 30 is a top view of the adapter of FIG. 29;
FIG. 31 is a front view of the adapter of FIG. 29;
FIG. 32 is a rear view of the adapter of FIG. 29;
FIG. 33 is a perspective view of the adapter being mated with the valve system of FIG. 17;
FIG. 34 is a cross sectional view of the adapter of FIG. 29;
FIG. 35 is a cross sectional view of the adapter of FIG. 29 being mated with the valve system of FIG. 17;
FIG. 36 is a partially exploded view of a bonded collar adapter for a port of the valve system of FIG. 17;
FIG. 37 is a cross sectional view of the collar of FIG. 36 being connected to an irrigation port of the valve system of FIG. 17;
FIG. 38 is a perspective view of the collar of FIG. 36 being connected to an irrigation port of the valve system of FIG. 17 with a irrigation syringe being removed from the port; and
FIG. 39 is a perspective view of the collar of FIG. 36 being connected to an irrigation port of the valve system of FIG. 17 with an irrigation syringe being connected to the port.

### DETAILED DESCRIPTION OF EXEMPLARY EMBODIMENTS

The exemplary embodiments of the valve system and methods of use disclosed are discussed in terms of medical systems inserted with a body of a subject for the administration of fluids, and more particularly, in terms of a valve system, having multiple ports, which is manipulated to establish fluid communication with a passageway of a nasogastric tube to avoid leakage of intestinal fluids and minimize disease propagation. It is envisioned that the present disclosure finds application for the removal of fluids from a body of the subject, such as aspirating fluids from the body or to introduce nutrients, supplements, medicines, etc. to the body. It is further envisioned that the valve system may be used with nasogastric tubing to decompress the contents of the subject's stomach and facilitate removal of accumulated fluids, blood, etc. from the GI tract due to disease, intestinal obstruction, bleeding ulcers and paralytic ulcers. It is contemplated that the valve system may be used with nasogastric tubing for abdominal surgery to keep the stomach vacant of fluid and postoperatively to prevent complications, such as, decreased gastrointestinal function. It is further contemplated that the valve system finds application in protecting gastric suture lines, preventing and treating paralytic ileus, treating drug overdoses, lavage, as well as other conditions that affect the GI tract. A practitioner may employ such a valve system in hospitals, nursing homes, care facilities, etc.

In the discussion that follows, the term "proximal" will refer to the portion of a structure that is closer to a practitioner, while the term "distal" will refer to the portion that is further from the practitioner. As used herein, the term "subject" refers to a human patient or other animal having fluids administered therewith, including removal and introduction as discussed herein. According to the present disclosure, the term "practitioner" refers to a doctor, nurse, or other care provider utilizing the valve system with medical tubing, and may include support personnel.

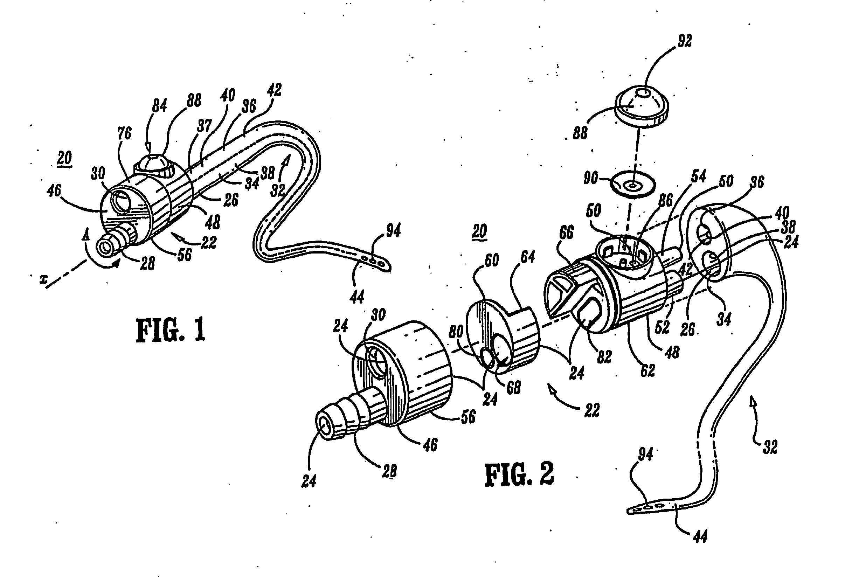

Reference will now be made in detail to the exemplary embodiments of the present disclosure, which are illustrated in the accompanying figures. Figs. 1-20 show an automatic valve system as described in WO 2004/039286. Turning now to the figures wherein like components are designated by like reference numerals throughout the several views and initially to FIGS. 1 and 2, there is illustrated a prior art nasogastric valve system 20.

Nasogastric valve system 20 includes a valve connector 22 that defines a longitudinal axis x and includes a second portion 24 of a first passageway 26. Valve connector 22 further includes a suction port 28 and an introduction port 30 that are spaced apart and in substantially parallel alignment. Suction port 28 and introduction port 30 are manipulable to establish fluid communication between portion 24 of first passageway 26 and suction port 28 or introduction port 30. This configuration avoids leakage of intestinal fluids and minimizes disease propagation, as will be discussed herein.

The component portions of valve connector 22, which may be disposable, are fabricated from materials suitable for nasogastric tubing applications for the administration of fluids with a subject including removal and introduction. These materials may include suitable medical grade, flexible, semi-rigid and rigid plastic materials, which may incorporate polyvinylchloride (PVC), silicone, etc., as well as medical grade metals, such as stainless steel and aluminum, depending on the particular nasogastric tubing application and/or preference of a practitioner. One skilled in the art, however, will realize that other materials and fabrication methods suitable for assembly and manufacture, in accordance with the present disclosure, also would be appropriate.

Nasogastric valve system 20 includes a flexible nasogastric tube 32 that has a fluid lumen 34 and a vent lumen 36. Fluid lumen 34 and vent lumen 36 are disposed in a side-by-side, parallel relationship and extend from a proximal end 37 to a distal end 44 of nasogastric tube 32. It is contemplated that nasogastric tube 32 may be monolithically formed or, alternatively, fluid lumen 34 and vent lumen 36 may be separately formed and integrally joined thereafter. It is further contemplated that fluid lumen 34 and vent lumen 36 may not be attached.

Fluid lumen 34 is configured to aspirate fluids from a GI tract of the subject (not shown) or to introduce nutrients, supplements, medicines, etc. to the subject. Vent lumen 36 is configured to regulate the amount of suction and flow during aspiration.

The component portions of nasogastric tube 32, which may be disposable, are fabricated from materials suitable for nasogastric tubing applications for the administration of fluids with a subject including removal and introduction. These materials may include suitable medical grade, flexible and semi-rigid plastic materials, which may incorporate polyvinylchloride (PVC), silicone, etc., as well as medical grade flexible metal structure, depending on the particular nasogastric tubing application and/or preference of a practitioner. One skilled in the art, however, will realize that other materials and fabrication methods suitable for assembly and manufacture, in accordance with the present disclosure, also would be appropriate.

Fluid lumen 34 defines a first portion 38 of first passageway 26. Vent lumen 36 defines a first portion 40 of a second passageway 42. First passageway 26 and second passageway 42 fluidly communicate adjacent a distal end 44 of nasogastric tube 32. It is envisioned that first passageway 26 and second passageway 42 do not fluidly communicate apart from distal end 44. Alternatively, first passageway 26 and second passageway 42 may fluidly communicate within valve connector 22 via appropriate structure, such as, for example, a connecting cavity, opening, etc. that facilitates communication therebetween.

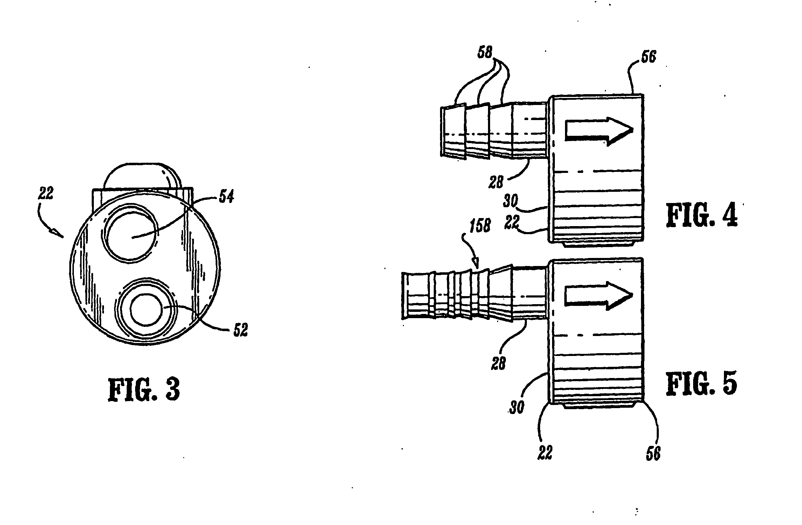

Valve connector 22 has a first end, such as, for example, proximal end 46 and a second end, such as, for example distal end 48. Referring to FIG. 3, distal end 48 includes a fluid port 52 and a vent port 54. Distal end 48 is attached to proximal end 37 of nasogastric tube 32 such that fluid port 52 and vent port 54 are received by fluid lumen 34 and vent lumen 36, respectively. Fluid port 52 and vent port 54 slidably engage respective interior surfaces of fluid lumen 34 and vent lumen 36 in a frictional interference fit to maintain a fluid sealing engagement between valve connector 22 and nasogastric tube 32. Valve connector 22 includes second portion 24 of first passageway 26 and a second portion 50 of second passageway 42.

Referring to FIGS. 4-7, valve connector 22 has a rotatable cylindrical outer cap 56 that includes suction port 28 and introduction port 30. Cap 56 is configured to facilitate manipulation of suction port 28 and introduction port 30 for establishing fluid communication with second portion 24 of first passageway 26. Cap 56 may have various cross-sectional configurations such as, for example, rectangular, polygonal, etc. to facilitate manipulation thereof. It is envisioned that cap 56 may be variably dimensioned with regard to, for example, diameter, length, etc. according to the requirements of a particular application.

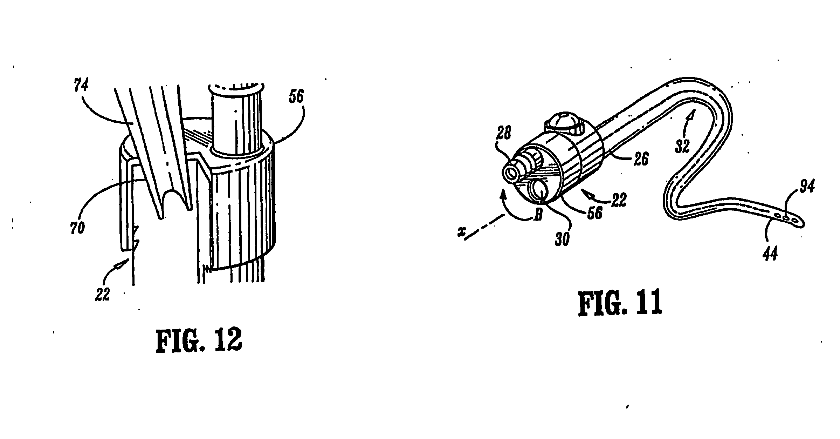

Cap 56 is manually rotated by the practitioner, in the direction shown by arrow A (counter clockwise) in FIG.1, to establish fluid communication between suction port 28 and first passageway 26 for aspirating fluids through fluid lumen 34. Alternatively, cap 56 is manually rotated by the practitioner, in the direction shown by arrow B (clockwise) in FIG. 11, to establish fluid communication between introduction port 30 and first passageway 26 for introducing nutrients, supplements, medicines, etc. to the subject. Cap 56 is rotatable through an angle of approximately 120 degrees to alternate fluid communication from suction port 28 to introduction port 30.

It is contemplated that cap 56 may be rotated clockwise and counter clockwise, in varying degrees of rotation through an angle up to and including 360 degrees, to establish fluid communication between suction port 28 or introduction port 30 and first passageway 26. It is further contemplated that cap 56 may be manipulated axially, angularly rotated relative to longitudinal axis x, etc. to establish fluid communication. It is envisioned that cap 56 may be rotated by mechanical, motorized, computerized, etc. devices to establish fluid communication with suction port 28 and introduction port 30, in accordance with the principles of the present disclosure.

Suction port 28 extends axially along longitudinal axis x and is configured for reception by suction tubing (not shown), which is connected to a source of suction (not shown), such as, for example, a vacuum pump, etc. Suction port 28 has a series of flanges 58, as shown in FIG. 4. Flanges 58 form a barb-like configuration to retain the suction tubing therewith. It is contemplated that the series of flanges 58 may be arranged in diameters that are uniform, increasing, decreasing, etc. to facilitate retention, according to the particular application. As shown in FIG. 5, an alternate embodiment of suction port 28 is shown, which includes flanges 158 that are arranged in an order of decreasing diameter.

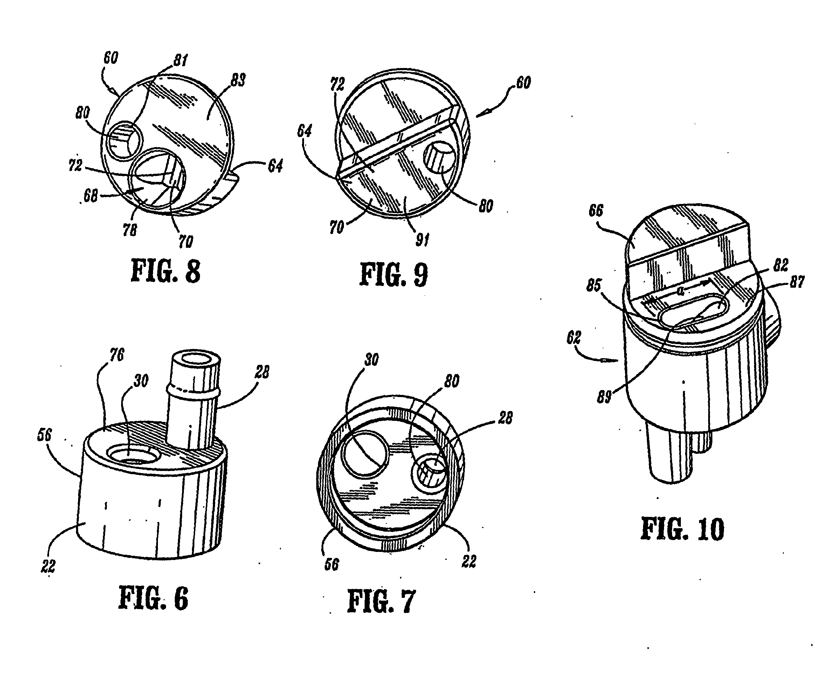

Referring to FIGS. 8-10, valve connector 22 includes a part 60 and a body 62. Part 60 is disposed within cap 56 and has a stepped portion 64 that is configured to engage and fit with a correspondingly configured stepped portion 66 of body 62. Part 60 is fabricated from an elastomeric material such as, for example, rubber, etc. and configured to facilitate manipulation of cap 56 to establish fluid communication between suction port 28 or introduction port 30 and first passageway 26. Cap 56 rotates relative to part 60 to align suction port 28 or introduction port 30 with first passageway 26 as desired, and will be discussed further below. The elastomeric material of part 60 enables sealing of first passageway 26 during fluid communication. It is envisioned that part 60 may be fabricated from less flexible plastics or suitable metals.

Introduction port 30 includes a normally closed valve 68 that is formed in part 60. Normally closed valve 68 includes an elastically deformable septum 70 having an elongate slit 72 formed through a thickness of septum 70. It is contemplated that all or only portions of septum 70 may be elastically deformable.

Septum 70 is elastically deformable such that a cannula 74 (FIG. 12) is engageable with elongate slit 72 to establish fluid communication between cannula 74 and first passageway 26 for introducing nutrients, supplements, medicines, etc. to the subject. Septum 70 has an angular orientation relative to longitudinal axis x to facilitate passing cannula 74 through slit 72. It is envisioned that septum 70 may be oriented at various angular orientations relative to longitudinal axis x, such as, for example, acute, perpendicular, etc. A feeding pump or the like may be introduced with introduction port 30 via septum 70 for constant or intermittent feeding of the subject.

Septum 70 is recessed relative to an outer surface 76 of valve connector 22. Valve connector 22 and normally closed valve 68 cooperate to define a recessed cylindrical cavity 78. It is contemplated that valve connector 22 or normally closed valve 68 may individually define cavity 78. Cavity 78 includes septum 70.

Part 60 includes a suction opening 80. Suction opening 80 facilitates communication between suction port 28 and first passageway 26. A raised lip 81 is circumferentially disposed, on a surface 83 of part 60, about suction opening 80. Surface 83 is configured for abutting engagement with the interior surface of cap 56 such that raised lip 81 facilitates sealing and prevents leakage of nasogastric valve system 20.

For example, cap 56 is manually rotated, in the direction shown by arrow A (counter clockwise) in FIG. 1, to establish fluid communication between suction port 28 and first passageway 26. Raised lip 81 is snug fit with the opening of suction port 28 in cap 56 to facilitate seating of cap 56 with part 60. This configuration prevents leakage from first passageway 26 during suction. It is contemplated that tactile feedback is provided to a practitioner via a snap, etc. fitting engagement of raised lip 81 with suction port 28, to indicate fluid communication is established between suction port 28 and first passageway 26. It is further contemplated that such tactile feedback indicates seating of suction port 28 with suction opening 80 and sealing of first passageway 26.

Alternatively, cap 56 is manually rotated, in the direction shown by arrow B (clockwise) in FIG. 11, to establish fluid communication between introduction port 30 and first passageway 26. In this orientation, raised lip 81 engages the interior surface of cap 56 to seal off suction opening 80. This configuration prevents vacuum leakage from suction port 28 during fluid communication between introduction port 30 and first passageway 26. It is envisioned that raised lip 81 may have various geometries, thickness, height, etc. according to the requirements of a particular application. It is further envisioned that raised lip 81 may be variously disposed about suction opening 80 such as, for example, intermittent, undulating, etc.

Normally closed valve 68 is disposed adjacent to suction opening 80 for alignment with an angled flow path 82 of body 62. Flow path 82 has a width a of sufficient dimension such that suction opening 80 and normally closed valve 68 of part 60 are concurrently positioned into alignment therewith. This configuration facilitates establishing fluid communication with suction port 28 or introduction port 30 upon rotation of cap 56 relative to part 60. As suction opening 80 and normally closed valve 68 are in alignment with first passageway 26, suction port 28 or introduction port 30 can be manipulated, as desired for removing or introducing fluids to the subject. It is contemplated that suction port 28 and insertion port 30 are manipulable to establish fluid communication between first passageway 26 and suction port 28 and insertion port 30 concurrently.

A raised lip 85 is disposed, on a surface 87 of body 62, about an opening 89 of angled flow path 82. Surface 87 is configured for abutting engagement with a surface 91 of part 60 such that raised lip 85 facilitates sealing and prevents leakage of nasogastric valve system 20. Raised lip 85 engages surface 91 to prevent leakage from first passageway 26 during use. It is envisioned that raised lip 85 may have various geometries, thickness, height, etc. according to the requirements of a particular application. It is further envisioned that raised lip 85 may be variously disposed about angled flow path 82 such as, for example, intermittent, undulating, etc.

Referring back to FIGS. 1 and 2, second portion 50 of second passageway 42 includes a relief port 84. Relief port 84 is disposed with valve connector 22 and protrudes from outer surface 76. Relief port 84 includes an opening 86 that communicates with second passageway 42 and vent lumen 36. Vent lumen 36, second passageway 42 and relief port 84 are configured to regulate the amount of suction and flow during aspiration. It is contemplated that relief port 84 may be employed to clear nasogastric tube 32. A cap 88 and valve 90 are mounted with relief port 84. Cap 88 defines an opening 92 that is configured to receive a cannula (not shown) or the like, which communicates with vent lumen 36. It is contemplated that relief port 84 may be connected to atmospheric air, venting source, etc. It is further contemplated that cap 88 may include a one-way valve, bi-directional valve, etc.

In operation, a valve system 20, similar to that described is provided for administration of fluids with a subject. The components of valve system 20 including valve connector 22 and nasogastric tube 32, similar to those described, are fabricated, properly sterilized and otherwise prepared for storage, shipment and use. Nasogastric tube 32 is manipulated such that fluid lumen 34 and vent lumen 36 receive fluid port 52 and vent port 54, respectively, as discussed. Thus, nasogastric tube 32 is attached to valve connector 22 so that second portion 24 and first portion 38 of first passageway 26 fluidly communicate. Second portion 50 and first portion 40 of second passageway 42 also fluidly communicate.

A practitioner introduces distal end 44 of nasogastric tube 32 through a nasal canal of a subject (not shown) via one of the nostrils. Distal end 44 is passed through the pharynx and down the esophagus into the GI tract. Distal end 44 can be passed into the duodenum, stomach, etc. depending on the particular application such as, for example, aspirating fluids, introduction for medication, feeding, etc. Several openings 94 are formed in distal end 44 that permit passage of gastric fluids, nutrients, medication, etc.

Cap 56 is rotated such that suction port 28 and introduction port 30 are manipulated to establish fluid communication between second portion 24 of first passageway 26 and suction port 28 or introduction port 30, as desired. Referring to FIG. 1, cap 56 is manually rotated by the practitioner, in the direction shown by arrow A (counter clockwise), to establish fluid communication between suction port 28 and first passageway 26 for aspirating fluids through fluid lumen 34 from the body of the subject. Raised lip 81 snugly fits with the opening of suction port 28 in cap 56, as described, providing tactile feedback to the practitioner that fluid communication is established and first passageway 26 is sealed. Valve system 20 may be connected to a collection vessel or the like to retain collected fluids.

Referring to FIG. 11, alternatively, cap 56 is manually rotated by the practitioner, in the direction shown by arrow B (clockwise), to establish fluid communication between introduction port 30 and first passageway 26 for injecting fluids. Raised lip 81 engages the interior surface of cap 56 to seal off suction opening 80, as described. In one embodiment, cannula 74 is inserted with normally closed valve 68, as discussed, to introduce nutrients, supplements, medicines, etc. to the body of the subject. Valve system 20 may be connected to a feeding pump or the like to provide constant or intermittent feeding. This configuration avoids leakage of intestinal fluids and minimizes disease propagation.

It is contemplated that nasogastric valve system 20 includes the necessary electronics and/or processing components to perform fluid measurement and analysis to facilitate diagnosis, treatment, etc. of a subject, as is known to one skilled in the art.

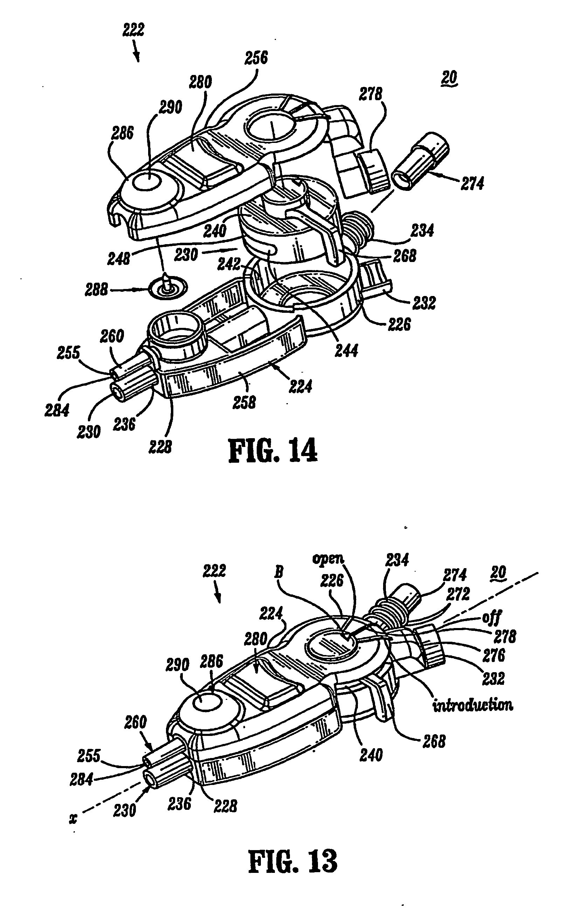

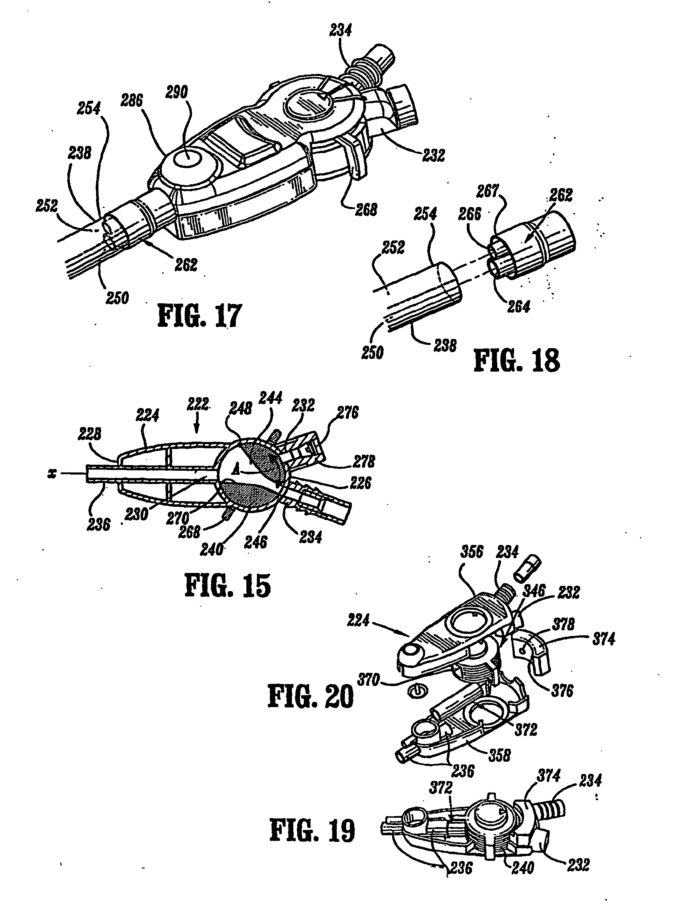

Referring to FIGS. 13-15, an alternate embodiment of nasogastric valve system 20, similar to that described above, is shown that includes a valve 222. Valve 222 includes a housing 224 that defines a first end 226 and a second end 228. Housing 224 also defines a longitudinal axis x and includes a first passageway 230 that extends therealong. Housing 224 includes a first port, such as, for example, introduction port 232 and a second port, such as, for example, suction port 234. Introduction port 232 and suction port 234 are disposed adjacent first end 226. It is contemplated that ports 232, 234 may be in parallel alignment, offset, angularly disposed, etc. Housing 224 further includes a third port, such as, for example, an attachment port 236 disposed adjacent second end 228. Attachment port 236 facilitates connection to a nasogastric tube 238 (FIG.17). First passageway 230 may have for example, a singular, branched, etc. configuration.

A valve member 240 is mounted for rotation within a cavity 242 of housing 224 relative to longitudinal axis x. Valve member 240 defines a portion 244 of first passageway 230 that includes a first opening 246 (FIG. 15) configured for alignment and sealed fluid communication with introduction port 232 (FIG. 15), in a first position, as will be discussed. First opening 246 is also configured for alignment and sealed communication with suction port 234 (FIG. 15), in a second position.

Referring to FIG. 15, portion 244 includes a second opening 248 (FIG. 14) having a greater relative dimension, as will be discussed, than first opening 246 and is configured to establish continuous fluid communication with first passageway 230. Valve member 240 is rotatable to establish sealed fluid communication between first opening 246 and introduction port 232, or alternatively, suction port 234, while maintaining a continuous sealed fluid communication between second opening 248 and attachment port 236. This configuration avoids leakage of intestinal fluids and minimizes disease propagation, as will be discussed herein.

Nasogastric valve system 20 includes flexible nasogastric tube 238 (FIGS. 17 and 18), similar to that described with regard to FIGS. 1-12, that has a fluid lumen 250 and a vent lumen 252. Fluid lumen 250 and vent lumen 252 are disposed in a side-by-side, parallel relationship and extend from a proximal end 254 to a distal end 44. Fluid lumen 250 defines a first portion of first passageway 230. Vent lumen 252 defines a portion of a second passageway 255. First passageway 230 and second passageway 255 (FIG. 13) fluidly communicate adjacent a distal end of nasogastric tube 238, similar to that shown in FIG. 2.

Referring to FIG. 14, housing 224 has a first section, such as, for example, top 256 and a second section, such as, for example bottom 258. Top 256 and bottom 258 are assembled together to enclose the components of valve 222. Top 256 and bottom 258 are ultrasonically bonded together, however, may be alternatively assembled employing adhesive, clips, etc.

An adapter 262 (FIG. 18) includes a fluid lumen 264 and a vent lumen 266 that are bonded to fluid lumen 250 and vent lumen 252 of nasogastric tube 238, as shown in FIG. 17, to provide a sealed fluid communication between valve 222 and tube 238. Adapter 262 facilitates connection of tube 238 and valve 222 and is configured such that a user can easily remove tube 238 from valve 222 for maintenance and replacement. Adapter 262 is bonded to tube 238 and includes a surface 267 at its periphery that bonds to the outer surface of tube 238 to provide strain relief. Adapter 262 may be ultrasonically bonded, adhered, monolithically formed with, etc. to nasogastric tube 238. Adapter 262 may be fabricated from a soft, semi-rigid or rigid material and include an oversized geometry, surface texturing, ribbing or additional material to enhance gripping and manipulation thereof.

Referring to FIGS. 13 and 18, second end 228 includes attachment port 236 and a vent port 260. Second end 228 is attached to adapter 262 such that attachment port 236 and vent port 260 are received by fluid lumen 264 and vent lumen 266, respectively. Attachment port 236 and vent port 260 slidably engage respective interior surfaces of fluid lumen 264 and vent lumen 266, facilitating corresponding fluid communication with lumens 250, 252, respectively, in a frictional interference fit to maintain a fluid sealing engagement between valve 222 and nasogastric tube 238.

Referring to FIGS. 14 and 15, valve member 240 is mounted within cavity 242 for rotation of first opening 246 to a plurality of positions. Valve member 240 includes a handle, such as, for example, selection knob 268 for manipulation thereof and to facilitate rotation of first opening 246, as shown by arrow A in FIG. 15, in a clockwise and counter-clockwise direction relative to longitudinal axis x. Selection knob 268 may be manipulated to rotate opening 246 into a first position, such as, for example, an open position (not shown).

In the open position, opening 246 is in alignment and sealed fluid communication with suction port 234. Indication to the user that opening 246 is in the open position is provided by visual indicia displayed from the outer surface of housing 224. The visual indicia includes a raised surface corresponding to the position of opening 246 and arrow B (FIG. 13) of valve member 240 that is directed to suction port 234. Alternative to the raised surface, depressions, mechanical detents, light emitting surface, etc. may be used. Valve 222 may also employ tactile and audible indicia, similar to the visual indicia used.

Opening 246 may be rotated into a second position, such as, for example, an introduction position (FIG. 15) such that opening 246 is in alignment and sealed fluid communication with introduction port 232. Indication to the user that opening 246 is in the introduction position is provided by visual indicia displayed from the outer surface of housing 224. Opening 246 may also be rotated into a third position, such as, for example, an off position (FIG. 13) such that opening 246 is not aligned with ports 232, 234 and fluid communication is prevented therebetween. Indication to the user that opening 246 is in the off position is provided by visual indicia displayed from the outer surface of housing 224

Referring to FIG. 15, valve member 240 has a solid core stopcock body including a bore 270 formed therethrough. Bore 270 flares from circular opening 246 to the wide oval configuration of second opening 248. This configuration accommodates rotation of valve member 240 while maintaining continuous sealed fluid communication between second opening 248 and first passageway 230 during manipulation of valve member 240. In an alternate embodiment as shown in FIGS. 19 and 20, valve member 240, mounted within a top 356 and a bottom 358 of a housing 324, defines a uniform bore 370. Tubing 372 is disposed within bore 370 for fluid communication with first passageway 230 (FIG. 15) and connects to attachment port 236. Tubing 372 extends to an opening 346 and is configured to facilitate continuous fluid communication with first passageway 230. Tubing 372 is flexible for rotation with valve member 240 to establish fluid communication with introduction port 232 and second port 234. A gasket 374, having openings 376 and 378 aligned with ports 232, 234 respectively, is mounted within housing 324 to facilitate fluid communication with first passageway 230. This configuration minimizes flow path volume and surface area.

Valve member 240 may have various configurations such as, for example, rectangular, polygonal, etc. to facilitate manipulation thereof. It is envisioned that valve member 240 may be variably dimensioned with regard to, for example, diameter, length, etc. according to the requirements of a particular application. Valve member 240 is fabricated from an elastometric material such as, for example, rubber, etc. and configured to facilitate manipulation thereof and establish fluid communication. Other semi-rigid and rigid materials are also contemplated.

It is contemplated that valve member 240 may be rotated clockwise and counter clockwise, in varying degrees of rotation through an angle up to and including 360 degrees, to establish fluid communication between ports 232, 234 and first passageway 230. It is further contemplated that valve member 240 may be manipulated axially, angularly rotated relative to longitudinal axis x, etc. to establish fluid communication. It is envisioned that valve member 240 may be rotated by mechanical, motorized, computerized, etc. devices to establish fluid communication with ports 232, 234, in accordance with the principles of the present disclosure.

Referring to FIGS. 13 and 15, opening 246 is releasably locked in the open position, introduction position and off position via an anti-tamper mechanism of valve 222. The anti-tamper mechanism includes a blocking member (not shown) that is connected to a release button 280 and engages valve member 240 to fix opening 246 in alignment with introduction port 232 in the introduction position; suction port 234 in the open position; and out of alignment in the off position. Such engagement between blocking member 240 prevents rotation of knob 268 and is deactivated before knob 268 can be rotated. Release button 280 and the blocking member include such known structure necessary to effect blocking and release of valve member 240 such as, for example, spring loaded, cam arrangements, pivoting structure, etc.

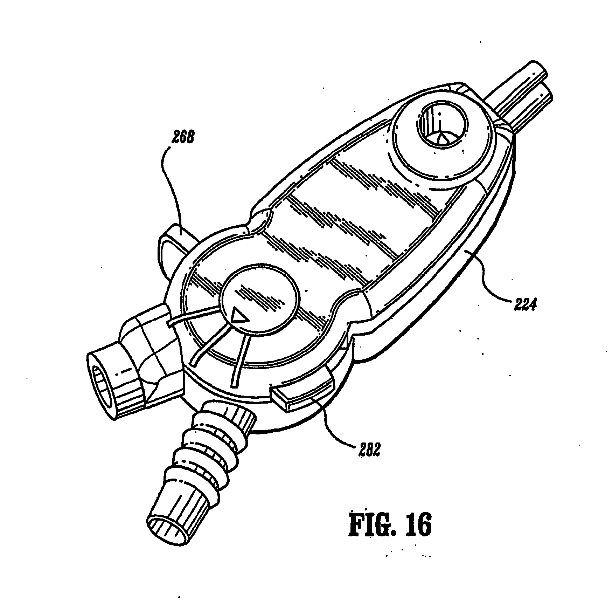

Housing 222 supports release button 280 that engages valve member 240 to release first opening 246 from alignment in a particular position. The practitioner depresses button 280 while manipulating knob 268. In an alternate embodiment, as shown in FIG. 16, an anti-tamper release button 282, similar to button 280 described, is disposed on a lateral portion of housing 224.

Referring to FIG. 13, suction port 234 extends at an angle slightly offset from longitudinal axis x and is configured for reception by suction tubing (not shown), which is connected to a source of suction (not shown), such as, for example, a vacuum pump, etc. Suction port 234 has a series of flanges 272 that form a barb-like configuration to retain the suction tubing therewith. It is contemplated that the series of flanges 272 may be arranged in diameters that are uniform, increasing, decreasing, etc. to facilitate retention, according to the particular application. Flanges 272 may be arranged in an order of decreasing diameter. Suction port 234 may include an enteral adapter 274 when continuous feeding is required. Adapter 274 is bonded to suction port 234, and may alternatively be adhered, monolithically formed, etc.

Introduction port 232 includes a normally closed valve 276 that is formed in a valve adapter 278 (FIGS. 14 and 15) bonded to port 232. Normally closed valve 276 (FIG. 15) includes an elastically deformable septum having an elongate slit formed through a thickness of the septum.

The septum is elastically deformable such that a cannula (not shown) is engageable therewith to establish fluid communication between the cannula and first passageway 230 for introducing nutrients, supplements, medicines, etc. to the subject. A feeding pump or the like may be introduced with introduction port 232 via the septum for constant or intermittent feeding of the subject.

Referring to FIGS. 14 and 17, second passageway 255 includes relief port 260. Relief port 260 includes an opening 284 that communicates with second passageway 255 and vent lumen 252 (FIG. 18). Vent lumen 252, second passageway 255 and relief port 260 are configured to regulate the amount of suction and flow during aspiration. It is contemplated that relief port 260 may be employed to clear nasogastric tube 238. An anti-reflux port 286 and anti-reflux valve 288 are mounted with housing 224 in fluid communication with relief port 260. Anti-reflux valve 288 prevents reflux from escaping through vent lumen 252 and regardless of the position of valve member 240, anti-reflux valve 288 is always operational.

Anti-reflux port 286 defines a membrane 290 that is configured to receive and permit passage of a cannula (not shown) or the like, for communicating with vent lumen 252. Anti-reflux port 286 allows the user to inject air into vent lumen 252 and establish a pressure activated buffer in vent lumen 252. It is contemplated that relief port 260 may be connected to atmospheric air, venting source, etc. It is further contemplated that cap 286 may include a one-way valve, bi-directional valve, etc.

In operation, a valve system 20, similar to that described with regard to FIGS. 13-20 in accordance with the principles of the present disclosure is provided for administration of fluids with a subject. The components of valve system 20 including valve 222 and nasogastric tube 238, similar to those described, are fabricated, properly sterilized and otherwise prepared for storage, shipment and use. Referring to FIGS. 15 and 17, nasogastric tube 238 and adapter 262 are manipulated such that fluid lumen 264 and vent lumen 266 receive attachment port 236 and relief port 260, respectively, as discussed. Thus, nasogastric tube 238 is attached to valve 222 to establish fluid communication with first passageway 230 and second passageway 255.

A practitioner introduces the distal end 44 of nasogastric tube 238 through a nasal canal of a subject (not shown) via one of the nostrils. The distal end 44 of nasogastric tube 238 is passed through the pharynx and down the esophagus into the GI tract. The distal end of nasogastric tube 238 can be passed into the duodenum, stomach, etc. depending on the particular application such as, for example, aspirating fluids, introduction for medication, feeding, etc.

Referring to FIGS. 15 and 17, knob 268 is manipulated for rotating opening 246 of valve member 240 to the open position, as confirmed by the visual indicia described above, to establish fluid communication between fluid lumen 250 and suction port 234. The open position setting facilitates suction through fluid lumen 250. Valve system 20 may be connected to a collection vessel or the like to retain collected fluids. Alternatively, an enteral feeding adapter 274 (FIG. 14) is attached to suction port 234 for continuous feeding.

Referring to FIG. 13, when medication introduction to fluid lumen 250 is desired, release button 280 is deactivated before knob 268 can be manipulated to the introduction position, as described above. The user depresses and holds button 280 while manipulating knob 268. Knob 268 is manipulated to rotate opening 246 (FIG. 15) to introduction port 232 (FIG. 15) in a clockwise direction, as shown by arrow A, and confirmed by the visual indicia. The user releases button 280 to activate the blocking member and fix valve member 240 in the introduction position. An irrigation tip syringe (not shown) is introduced through normally closed valve 276 to administer medication, etc. through fluid lumen 250 (FIG. 18). Normally closed valve 276 automatically closes upon removal of the syringe to check backflow of contaminating gastric fluids. Valve system 20 may be connected to a feeding pump or the like to provide constant or intermittent feeding.

When patient transportation is desired, the user depresses and holds button 280 and manipulates knob 268 to rotate opening 246 to the off position, as confirmed by the visual indicia, in preparation for patient transportation. The user releases button 280 to activate the blocking member and fix valve member 240 in the off position. The configuration of valve system 20 avoids leakage of intestinal fluids and minimizes disease propagation.

In addition to nasogastric tube 238, other single lumen tubes may be used with valve 222 of the present disclosure. Such tubes having a single lumen may be used with the valve 222 of the present disclosure for a nasogastric tube 238 or for another tube. Such single lumen tubes can be passed into the duodenum, stomach, etc. depending on the particular application such as, for example, aspirating fluids, introducing medication, feeding materials into the body (such as into the stomach), or draining materials and fluids from the body out of the stomach. One such single lumen tube is a PEG tube or a Percutaneous Endoscopic Gastrostomy single lumen tube. Such a single lumen PEG tube may be introduced through an abdominal wall and into the stomach for draining the stomach or feeding fluids into the stomach. Various other single lumen tubes exist and the present disclosure is not to be limited by any single lumen tubes.

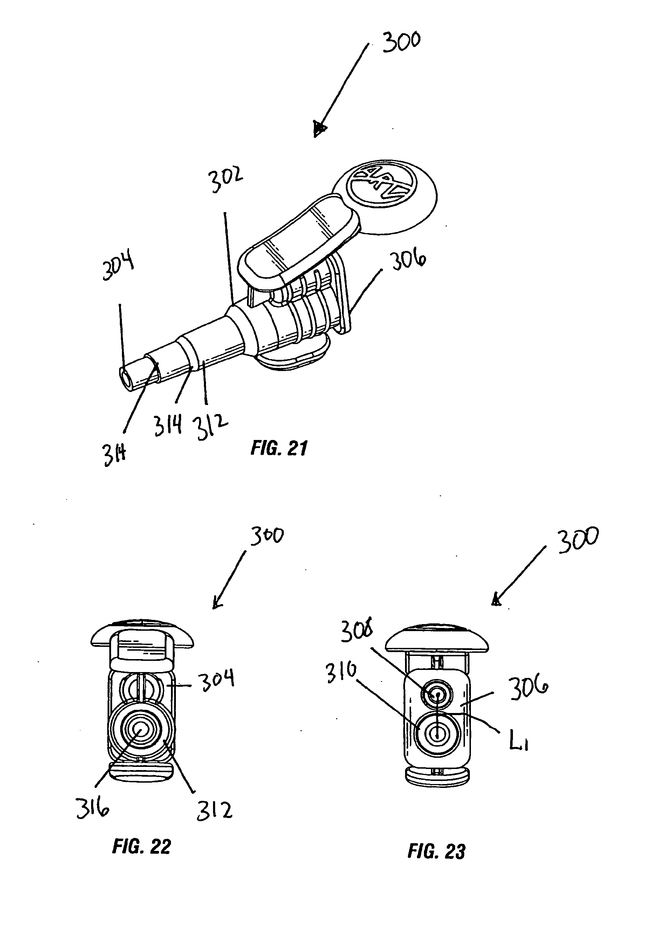

Referring now to Fig. 21, there is shown a fluid adapter 300 for the valve 222 of FIG. 14. The fluid adapter 300 has a housing 302 and is for connecting to the attachment port 236 and to the vent port 260 of the valve 222 shown in FIG. 14. The fluid adapter 300 has a distal side 304. This distal side 304 connects to a feeding tube (not shown) or another drainage tube having a single passageway or a single lumen. Specifically, the valve 222 of Fig. 14 may be desired to be connected to a single lumen tube for a variety of applications. One application may include using a single lumen stomach tube for feeding. Another is a single lumen stomach tube for draining the stomach. Such single lumen stomach tubes use a single lumen or passageway where simply no relief valve or vent lumen is needed. The present disclosure is not intended to be limited to any size or dimensions for single lumen tubes.

In one such application, the practitioner may not need any such vent port 260 or anti-reflux port 286 as shown in Fig. 14. In practice, the practitioner may, in order to use the valve 222, unduly search for a connector or another valve system or perform an amount of substantive work in order to replace the entire valve system or find a replacement valve to connect to the single lumen tube. In the instant application, the practitioner would not have to disturb an orientation of the valve 222. Instead, the practitioner simply uses the present fluid adapter 300. The present fluid adapter 300 blocks the vent port 260 of the valve 222, and a portion of the valve 222 in order to quickly and easily convert the valve 222 for a single lumen tube use for feeding or drainage. The present fluid adapter 300 also during use provides for a clean operation relative to the bedside setting of the patient whereas no or a negligible amount of fluid is spilled during the transfer in order to prevent soiling the bedside setting. This avoids leakage of intestinal fluids and minimizes disease propagation. Sometimes if another prior art adapter is located by the practitioner and that prior art adapter does not properly fit the valve 222, spillage could occur that can soil the bedside setting. Moreover, if the practitioner is searching for another relatively suitably sized adapter spillage may occur if they disturb an orientation of the valve 222 while searching. This may further promote spillage that can spoil or soil the bedside setting. The present single lumen fluid adapter 300 prevents such known detriments from occurring.

Referring now to Fig. 21, the fluid adapter 300 has a housing 302. The housing 302 has a distal end 304 and a proximal end 306. Referring now to the proximal end 306 of the fluid adapter 300, shown in Fig. 23, the adapter 300 has a first circular shaped opening 308 and a second shaped opening 310. The first opening 308 forms a first lumen that terminates at a distal end 304. The second opening 310 is separated from the first opening 308 by a distance "L1". The second opening 310 forms a second lumen. The second lumen extends through the housing 302 of the fluid adapter 300.

Referring now again to the distal end 304 of the housing 302 (shown in Figs. 21 and 22), the fluid adapter 300 has a neck portion 312. The neck portion 312 extends outward and has a number of flanges 314. The number of flanges 314 each has a differently sized or similar complementary diameter relative to the remainder of the flanges 314. The flanges 314 permit a sealing engagement with an inner surface of the feeding or drainage tube when the single lumen tube is placed over the neck portion 312 of the adapter 300. The single lumen tube need not be placed over all of the flanges 314. Alternatively, the single lumen tube may only be placed over one or some of the flanges 314 or an intermediate location of the neck portion 314 to ensure that the single lumen tube is connected to the neck portion 314. Referring now to Fig. 22, the neck portion 312 also has an opening 316. The opening 316 of the neck portion 312 mates with the single lumen of the feeding/drainage tube.

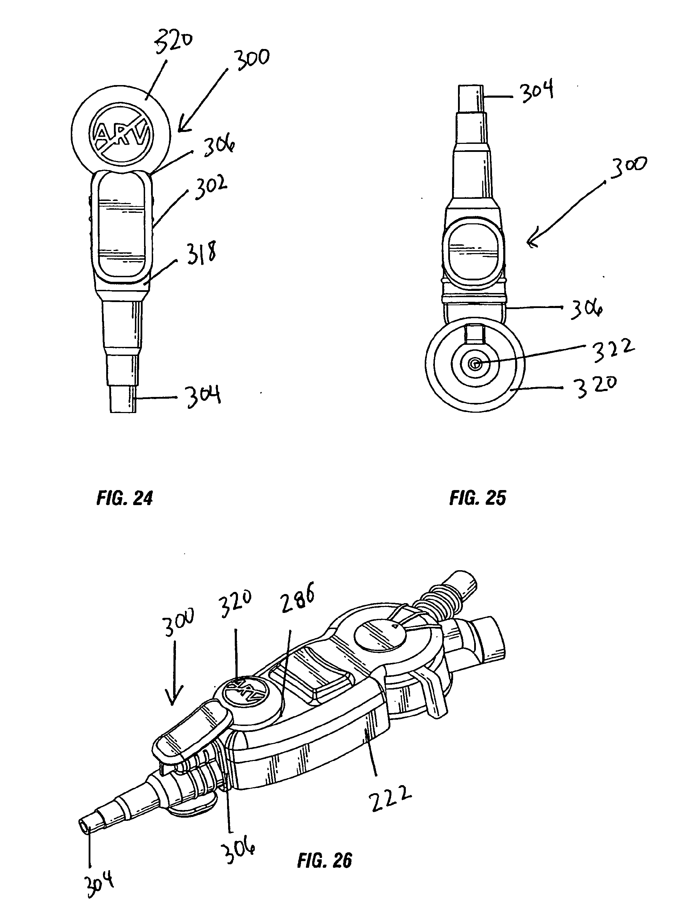

Referring now Fig. 24 there is shown a top side 318 of the housing 302. The fluid adapter 300 has a tab 320. The tab 320 hangs over from the proximal end 306 of the housing 302. The tab 320 extends over to cover over a portion of the valve 222. Referring now to Fig. 25, the tab 320 has a detent 322. The detent 322 is a male protrusion. The detent 322 occludes and optionally engages with an anti-reflux port 286 of the valve 222. The tab 320 prevents the practitioner from engaging the anti-reflux port 286 when the fluid adapter 300 is connected to the valve 222. This prevents the practitioner from toggling the anti-reflux port 286 by blocking it. The tab 320, thus, prevents the practitioner from engaging the anti-reflux port 286 even accidentally. Toggling anti-reflux port 286 when using a single lumen tube may cause a spray or spillage of fluids to occur. The present single lumen adapter 300 prevents any such toggling in operation. Fig. 26 shows the adapter 300 in a perspective view being connected to the valve 222. As can be seen, the tab 320 of the adapter 300 covers the anti-reflux port 286 to prevent the user from toggling the anti-reflux port 286. The tab 320 primarily occludes a portion of the valve 222, and further in another embodiment may lock the anti-reflux port 286 with the detent 322.

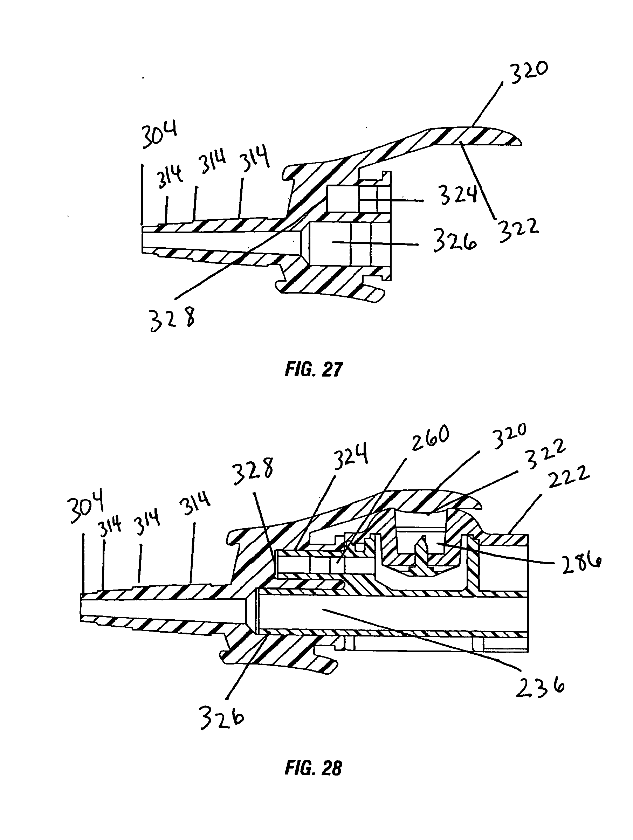

Referring now to Fig 27, there is shown a cross sectional view of the adapter 300. As can be seen from the cross sectional view, the adapter 300 has a first lumen 324. The first lumen 324 terminates a distance from first opening 308 at a distal end 328. Referring now to Fig. 28, the vent port 260 of the valve 222 engages the first lumen 324. The first lumen 324 terminates at the end 328 and thus closes the vent port 260. The adapter 300 thus prevents operation of the vent port 260. Referring again to Fig. 27, the fluid adapter 300 also has a second lumen 326. The second lumen 326 engages with the attachment port 236 of the valve 222 (shown in Fig. 28). The attachment port 236 permits fluid to traverse through the feeding/drainage tube having the single lumen. The fluid will pass through the second lumen 326 of the adapter 300 to the feeding/drainage tube. As can be understood from the figures and, as shown, the distal end 304 of the adapter 300 has a number of flanges 314. The number of flanges 314 each has a differently sized or similar complementary diameter relative to the remainder of the flanges 314. The flanges 314 permit a sealing engagement with an inner surface of the feeding/drainage tube having a single lumen with ease and repeatability.

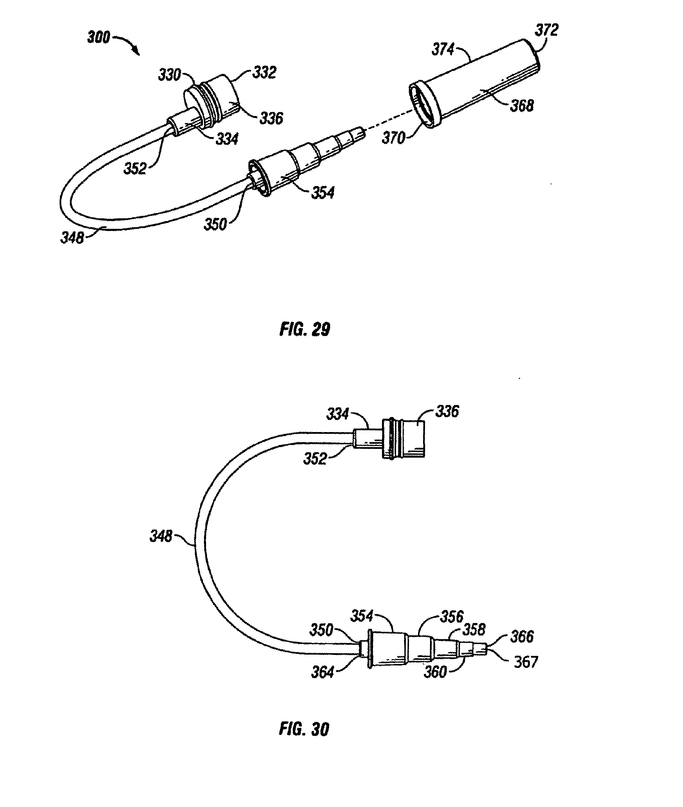

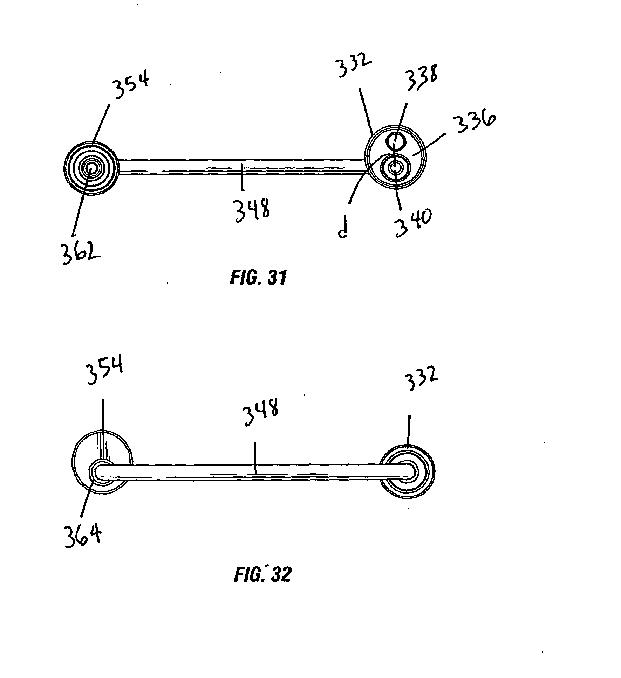

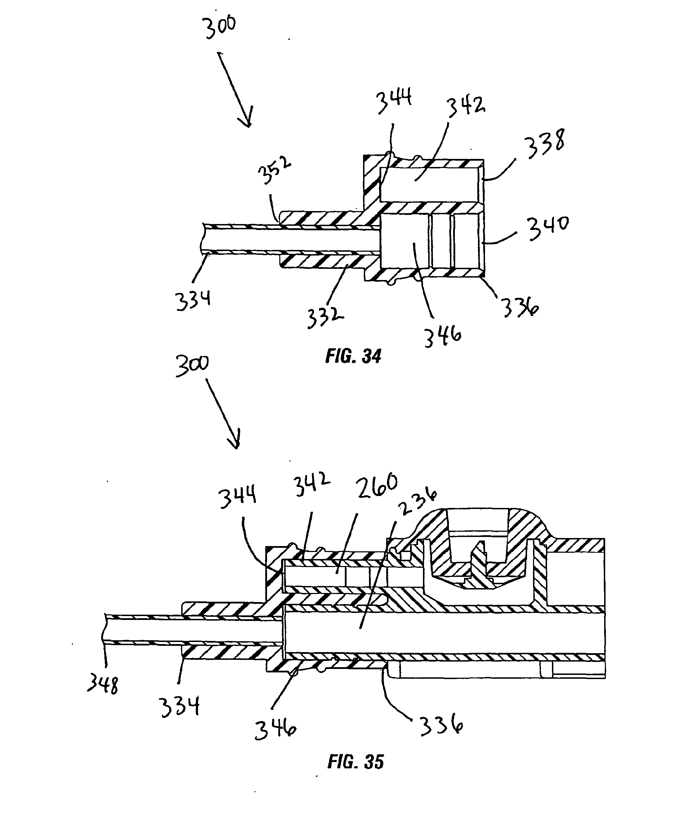

Referring now to Fig. 29, there is shown another alternative embodiment of the present adapter 300. As shown in Fig. 29, the adapter 300 has a first portion 330 with a housing 332. The housing 332 has a distal end 334 and a proximal end 336. Referring now to Fig. 31, the proximal end 336 of the housing 332 has a first circular opening 338 and a second circular opening 340 that is spaced away from the first circular opening 338 by a distance d. Similar to the embodiments shown in Fig. 21 through 28, (as shown in a cross sectional view in Fig. 34) the first circular opening 338 communicates with a first lumen 342 in the housing 332. The first lumen 342 has a terminal end 344 (Fig. 34). The first circular opening 338 and thus the lumen 342 engage with the vent port 260 of the valve 222 as shown in Fig. 35. The first lumen 342 with end 344 thus closes the vent port 260 and prevents any fluid or gas from the ambient or the feeding/drainage tube to traverse therethrough. Referring again to Fig. 34, the second circular opening 340 communicates with a second lumen 346. The second lumen 346 extends through the housing 332 of the adapter 300. The second lumen 346 extends from the distal end 334 to the proximal end 336 (of adapter 300).

Referring back now to Fig. 29, as can be seen the housing 332 of the adapter 300 communicates with an intermediate tubing 348. Specifically, the second lumen 346 (of the first portion 330 of the adapter 300 that is shown in Fig. 34) communicates with the intermediate tubing 348. The intermediate tubing 348 is a cylindrical tube. The intermediate tubing 348 is opened on a first distal end 350, and is also opened at an opposite proximal end 352 (Fig. 29). Referring to FIG. 34, as shown in cross section, the opening of the end 352 of tubing 348 communicates with the second lumen 346 of the housing 332 of adapter 300. As shown in FIG. 29, the opening of the other end 350 of the cylindrical tubing 348 communicates with a flanged member 354. The flanged member 354 is a resilient member having a number of flanges therearound. In Fig. 30, each of the flanges of the flanged member 354 has a reducing or differently sized diameter. As shown, the flanged member 354 has a first flange 356 having a first diameter. The flanged member 354 also has a second flange 358 having a second diameter that is less than the first diameter. The flanged member 354 further has a third flange 360 with a third diameter. The third diameter is less than the second diameter. In Fig. 31, the flanged member 354 further has a flange lumen 362 that extends through the flanged member 354. The intermediate tubing 348 is connected through the flanged lumen 362. The intermediate tubing 348 is also connected to the flanged member 354 at the proximal end 364 of the flanged member 354 as shown in Fig. 32. In one embodiment, the intermediate tubing 348 is connected to the flanged member 354 by an adhesive. The intermediate tubing 348 may be connected by other means such as by welding, by a solvent or a fastener.

Referring now to Fig. 30, the flanged member 354 also has a distal end 366. The distal end 366 terminates at an opening 367. The opening 367 communicates with the flanged lumen 362 in a sealed manner to permit fluids to traverse therethrough.

Referring now again to FIG. 29, there is shown a perspective view of the adapter 300. The adapter 300 further has a cover 368. The cover 368 is a cylindrical shaped member. The cylindrical shaped cover 368 extends over the flanged member 354 as shown by a dotted line. The cover 368 further is opened at a first proximal side 370 and a second distal side 372. The cover 368 further has a resilient exterior surface 374. The resilient exterior surface 374 protects the adapter 300 until use and is shortly discarded thereafter.

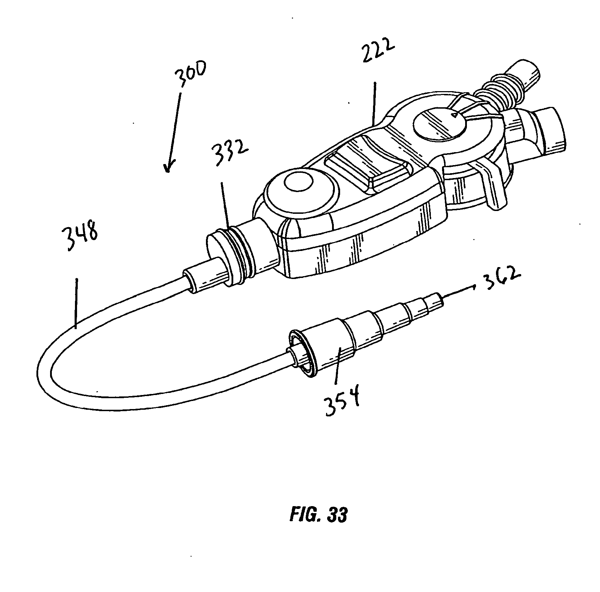

In operation, the feeding/drainage tube is engaged to the flanged member 354 by sliding the feeding/drainage tube over the number of flanges 356, 358, and 360 of the flanged member 354. The proximal end 364 of the flanged member 354 will be connected to the intermediate tubing 348. Thus, the length of the intermediate tubing 348 is convenient for the practitioner as the length permits an amount of slack between the valve 222 and the feeding/drainage tube so as to not disturb an orientation of the feeding/drainage tube relative to the patient. In operation, as shown in Figs. 33 and 35 with the slack, the practitioner can manipulate the adapter 300 in order to place the vent tube 260 into the first circular opening 338 (Fig. 35). In this manner, the first lumen 342 that terminates at a terminal end 344 connects to the vent port 260 of the valve 222 and closes the vent port 260 (Fig. 35). In operation with the slack, the practitioner simultaneously manipulates the adapter 300 in order to place the attachment port 236 of the valve 222 into the second circular opening 340. In this manner, the second lumen 346 (that extends through the housing 332 of the adapter 300 to the distal end 334 of the housing 332) connects to the attachment port 236 of the valve 222 and permits fluid to traverse from the valve 222 through the attachment port 236 and through the adapter 300 (Fig. 35).

Fig. 33 shows that the fluid may traverse from the adapter 300 through the cylindrical intermediate tubing 348. Notably, the cylindrical intermediate tubing 348 of the single lumen adapter 300 is translucent. The translucent property permits the practitioner to easily inspect the contents thereof, and gauge when an appropriate time when the single lumen adapter 300 may be engaged or disengaged from the valve 222 and/or feeding/drainage tube to avoid spillage or soiling the surrounding areas.

The fluid then traverses from the cylindrical intermediate tubing 348 to the flanged member 354 and through the flanged lumen 362. The fluid will then traverse through the flanged member 354 and into the feeding/drainage tube that is connected in a sealed manner thereto and into the patient for feeding and/or drainage.

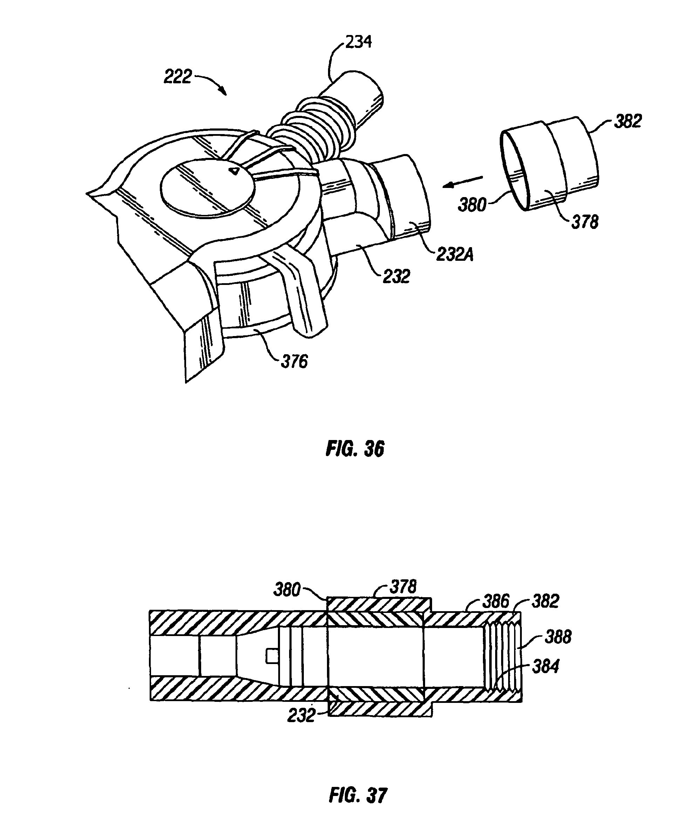

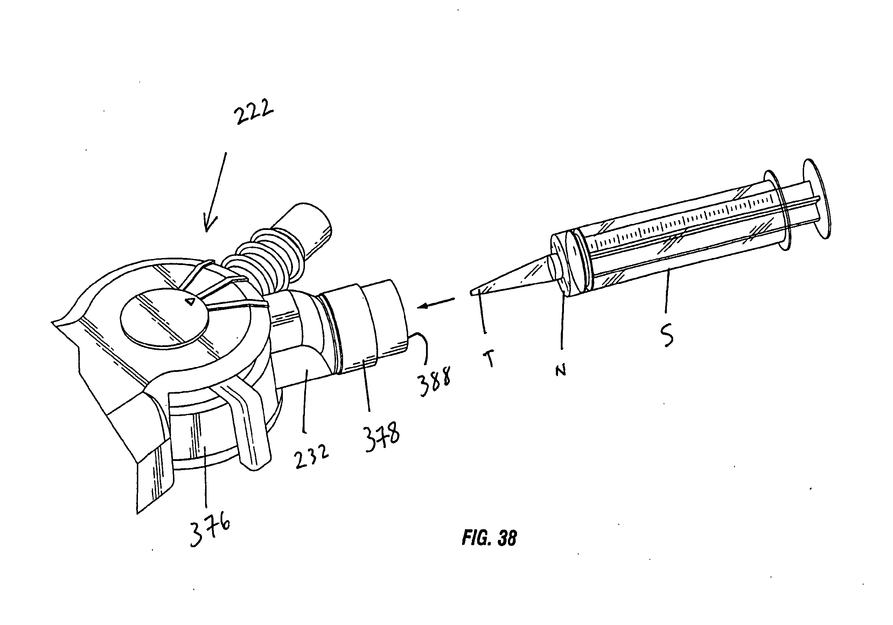

Referring now to Fig. 36, there is shown another exemplary embodiment of the present disclosure. Referring to Fig. 36, there is shown the valve 222. Referring to a first side 376 of the valve 222, there is shown the introduction port or irrigation port 232. The irrigation port 232 of the valve 222 is for (in some uses) engagement with an irrigation syringe S (Fig. 38). In some embodiments, the valve 222 has the irrigation port 232 being made from a resilient material such as a thermoplastic.

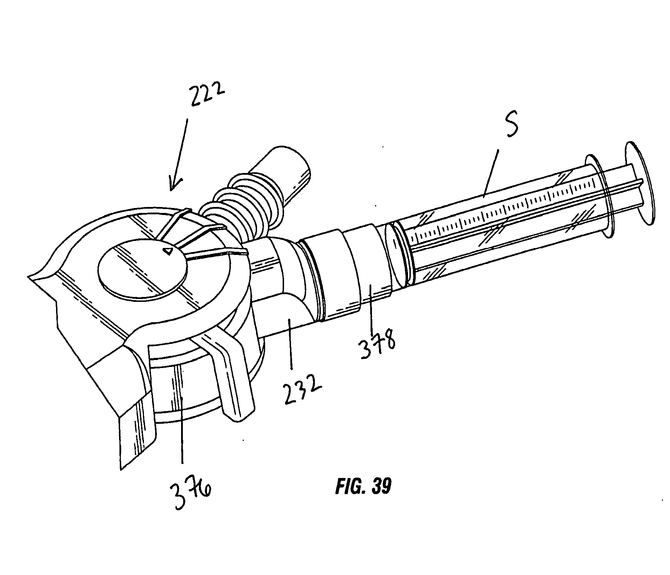

The resilient material, although suitable for use for mating with an irrigation syringe S may not perfectly mate with all syringes having a number of differently sizes. As can be understood, syringes have a neck portion N with a tip T that extends from the neck portion N. Due to the variety and number of differently sized syringes S, there may be a perception that the syringe S does not properly fit onto the irrigation port 232. Additionally, the surface to surface contact between the neck N of the syringe S and an inner surface of the irrigation port 232 may have an amount of surface adhesion. In order to remedy this concern, the present valve 222 has a bonded irrigation port adapter 378. The syringe also has a taper length L3 (Fig. 38). This taper length from a tip T to the neck N may vary due to the size of the many different syringes that can be used with the present disclosure. Accordingly, taper length L3 can vary depending on the size of the syringe. Thus, the bonded irrigation port adapter 378 ensures that the bonded irrigation port adapter 378 can press fit and hold the syringe S at any location on the taper length L3 for a number of various syringe sizes (Fig. 39).

Referring again to Fig. 36, the bonded irrigation port adapter 378 (or "collar") is generally cylindrical in shape. The bonded irrigation port adapter 378 has a distal most opening 380 and a proximal most opening 382. Referring now to a cross sectional view of the bonded irrigation port adapter 378 shown in FIG. 37, the bonded irrigation port adapter 378 has an inner surface 384 and an outer surface 386. The inner surface 384 has one or more protrusions 388. In one embodiment, the inner surface 384 may have three protrusions 388. The distal most opening 380 of the bonded irrigation port adapter 378 is connected to the irrigation port 232 of the valve 222. The bonded irrigation port adapter 378 may be connected by a number of different methods. In one embodiment, the bonded irrigation port adapter 378 may be adhered to the irrigation port 232. In another embodiment, bonded irrigation port adapter 378 may be ultrasonically bonded, solvent bonded, RF bonded or adhered to the irrigation port 232 or fastened by a connector. As shown in the partially exploded view of Fig. 36, the irrigation port 232 may have an end 232A with a reduced diameter. The reduced diameter accommodates a size of the bonded irrigation port adapter 378. The reduced diameter permits the irrigation port 232 having the bonded irrigation port adapter 378 to have one uniform width across a length of the irrigation port 232 when the adapter 378 is connected to the valve 222 (when assembled).

The bonded irrigation port adapter 378 is made from a soft spongy material that is flexible. In one embodiment, the bonded irrigation port adapter 378 is made from a flexible polyvinyl chloride material or another flexible thermoplastic, polyurethane, or a composite material. Alternatively, the bonded irrigation port adapter 378 can be made from a silicone or another material. In Fig. 38, the bonded irrigation port adapter 378 has a number of protrusions 388 and the protrusions 388 are on an inner periphery 384 of the bonded irrigation port adapter 378. The protrusions 388 flexibly receive the taper length L3 of the syringe S. Referring now to Fig. 39, the flexible protrusions 388 can deform and engage at any point along the taper length L3 of the irrigation syringe S. In this manner, the protrusions 388 of the bonded irrigation port adapter 378 hold the irrigation syringe S in the irrigation port 232 and in place relative to the valve 222 in order to introduce the tip T into the bonded irrigation port adapter 378. Sealing is intended to take place at any location along the taper length L3 along the syringe S and including the neck N. In this manner, the irrigation syringe S can release the contents of the irrigation syringe S through the irrigation port 232 and through the valve 222 to the feeding/drainage tube.

It will be understood that various modifications may be made to the embodiments disclosed herein. Therefore, the above description should not be construed as limiting, but merely as exemplification of the various embodiments. Those skilled in the art will envision possible other modifications.

## Claims

1. A fluid adapter (300) for connection between a valve and a tube having a single lumen, the fluid adapter comprising:
a housing (302) having a distal end (304) and a proximal end (306);
wherein said proximal end (306) has a first opening (326) connecting to a first lumen being disposed through said housing;
wherein said proximal end (306) has a second opening (324) being separated by said first port by a distance, said second opening being a close ended port connecting to a second lumen; and
wherein said distal end has said first lumen connected to a single outlet (304), said outlet connecting to the tube having the single lumen,
**characterized in that** said housing (302) further comprises a tab (320), said tab (320) being releasably connected over a secondary port (286) of the valve (222) and wherein said tab (320) is configured to occlude the secondary port to prevent the user from toggling the secondary port and said housing is configured to be releasably connected to the valve.

2. The fluid adapter (300) of claim 1 for connection between a valve having a plurality of ports and a tube, the tube having a single lumen, wherein said housing connects to an intermediate tube (348); and wherein said intermediate tube (348) is connected to the tube to permit fluid to traverse through the tube and the valve.

3. The fluid adapter of claim 2, wherein said intermediate tube is connected to a member (304) having a distal end with a neck portion configured to permit the tube having the single lumen to fasten over said neck portion.

4. The fluid adapter of claim 3, wherein said neck portion (304) of said member comprises a plurality of flanges (314).

5. The fluid adapter of claim 4, wherein said plurality of flanges (314) are configured to form a barb-like configuration to retain the tube thereon in a sealed manner.

6. The fluid adapter of claim 5, wherein said plurality of flanges (314) has at least one with a first diameter and at least another flange with a second diameter with said first diameter and said second diameter being differently sized diameters.

7. The fluid adapter of claim 5, wherein said plurality of flanges (314) are sized to facilitate retention of the tube in a sealed manner.

8. The fluid adapter of claim 5, wherein said plurality of flanges (314) has at least one with a first diameter and at least another flange with a second diameter with said first diameter and said second diameter being differently sized diameters to facilitate retention of the tube.

9. The fluid adapter of claim 2, wherein said housing is generally cylindrical and sized to be manipulated by hand.

10. The fluid adapter of claim 9, wherein said intermediate tube (348) has a length,
wherein said length is suitable to rotate the valve being connected to the housing without disengaging said housing from the valve.

11. The fluid adapter of claim 1, wherein said first lumen is generally cylindrical.

12. The fluid adapter of claim 1, wherein said second lumen is generally cylindrical and terminates at an end.

13. The fluid adapter of claim 1, wherein said housing is made from a flexible material.

14. The fluid adapter of claim 1, wherein at least one of said first lumen and said second lumen has a first size being relatively smaller than another of said first lumen and said second lumen to accommodate the plurality of ports of the valve.

15. The fluid adapter of claim 14, wherein at least one lumen terminates at an end to close at least one of the plurality of ports of the valve, said closed port being a vent port.

16. The fluid adapter of claim 3, wherein said intermediate tube (348) is connected to a flanged member having a plurality of flanges (314) to permit the tube having the single lumen to fasten over the plurality of flanges.

17. The fluid adapter of claim 2, wherein said intermediate tube (348) is transparent and configured to permit a visual inspection thereof.

18. The fluid adapter of claim 1, wherein said housing is made from a thermoplastic.

19. The fluid adapter of claim 1, wherein said housing is made from a material selected from the group consisting of a phthalate free polyvinyl chloride, a HDPE plastic, a PP plastic, and any combination thereof.

20. The fluid adapter of claim 1, wherein at least one opening has a compression fit, and wherein another opening has a relatively looser fit relative to said compression fit.

21. The fluid adapter of claim 1, wherein said single outlet (304) has a neck portion with a plurality of flanges (314), said plurality of flanges forming a barb like configuration to connect with the tube in a sealed manner.

## Patentansprüche

1. Fluidadapter (300) zur Verbindung zwischen einem Ventil und einem Schlauch mit einem einzigen Lumen, wobei der Fluidadapter Folgendes umfasst:
ein Gehäuse (302) mit einem distalen Ende (304) und einem proximalen Ende (306);
wobei das proximale Ende (306) eine erste Öffnung (326) aufweist, die mit einem durch das Gehäuse ausgebildeten ersten Lumen verbunden ist;
wobei das proximale Ende (306) eine zweite Öffnung (324) aufweist, die durch einen Abstand von dem ersten Anschluss getrennt ist, wobei die zweite Öffnung ein Anschluss mit einem geschlossenen Ende ist, der mit einem zweiten Lumen verbunden ist; und
wobei das distale Ende das erste Lumen aufweist, das mit einem einzigen Auslass (304) verbunden ist, wobei der Auslass den Schlauch mit dem einzigen Lumen verbindet,
**dadurch gekennzeichnet, dass** das Gehäuse (302) weiterhin einen Ansatz (320) umfasst, wobei der Ansatz (320) über einen zweiten Anschluss (286) des Ventils (222) lösbar verbunden ist, und wobei der Ansatz (320) dazu konfiguriert ist, den zweiten Anschluss zu verschließen, um zu verhindern, dass der Benutzer den zweiten Anschluss umschaltet, und das Gehäuse ist dazu konfiguriert, lösbar mit dem Ventil verbunden zu werden.

2. Fluidadapter (300) nach Anspruch 1 zur Verbindung zwischen einem Ventil mit mehreren Anschlüssen und einem Schlauch, wobei der Schlauch ein einziges Lumen aufweist, wobei das Gehäuse mit einem Zwischenschlauch (348) verbunden ist und wobei der Zwischenschlauch (348) mit dem Schlauch verbunden ist, um zu gestatten, dass Fluid den Schlauch und das Ventil durchquert.

3. Fluidadapter nach Anspruch 2, wobei der Zwischenschlauch mit einem Glied (304) verbunden ist, der ein distales Ende mit einem Halsteil aufweist, das dazu konfiguriert ist, zu gestatten, den Schlauch mit dem einzigen Lumen auf dem Halsteil zu befestigen.

4. Fluidadapter nach Anspruch 3, wobei der Halsteil (304) des Glieds mehrere Flansche (314) umfasst.

5. Fluidadapter nach Anspruch 4, wobei die mehreren Flansche (314) dazu konfiguriert sind, eine widerhakenartige Konfiguration zu bilden, um den Schlauch abgedichtet daran zu halten.

6. Fluidadapter nach Anspruch 5, wobei die mehreren Flansche (314) mindestens einen mit einem ersten Durchmesser und mindestens einen anderen mit einem zweiten Durchmesser umfassen, wobei der erste Durchmesser und der zweite Durchmesser verschieden bemessene Durchmesser sind.

7. Fluidadapter nach Anspruch 5, wobei die mehreren Flansche (314) dazu bemessen sind, ein abgedichtetes Festhalten des Schlauchs zu erleichtern.

8. Fluidadapter nach Anspruch 5, wobei die mehreren Flansche (314) mindestens einen mit einem ersten Durchmesser und mindestens einen anderen mit einem zweiten Durchmesser umfassen, wobei der erste Durchmesser und der zweite Durchmesser verschieden bemessene Durchmesser sind, um das Festhalten des Schlauchs zu erleichtern.

9. Fluidadapter nach Anspruch 2, wobei das Gehäuse allgemein zylindrisch und zur Betätigung von Hand bemessen ist.

10. Fluidadapter nach Anspruch 9, wobei der Zwischenschlauch (348) eine Länge aufweist, wobei die Länge dazu geeignet ist, das mit dem Gehäuse verbundene Ventil ohne Ausrücken des Gehäuses aus dem Ventil zu drehen.

11. Fluidadapter nach Anspruch 1, wobei das erste Lumen allgemein zylindrisch ist.

12. Fluidadapter nach Anspruch 1, wobei das zweite Lumen allgemein zylindrisch ist und an einem Ende abschließt.

13. Fluidadapter nach Anspruch 1, wobei das Gehäuse aus einem flexiblen Material hergestellt ist.

14. Fluidadapter nach Anspruch 1, wobei das erste Lumen und/oder das zweite Lumen eine erste Größe aufweist/aufweisen, die im Verhältnis kleiner ist als eine andere des ersten Lumens und des zweiten Lumens, um den mehreren Anschlüssen des Ventils Rechnung zu tragen.

15. Fluidadapter nach Anspruch 14, wobei mindestens ein Lumen an einem Ende abschließt, um mindestens einen der mehreren Anschlüsse des Ventils zu schließen, wobei der geschlossene Anschluss ein Lüftungsanschluss ist.

16. Fluidadapter nach Anspruch 3, wobei der Zwischenschlauch (348) mit einem Flanschglied mit mehreren Flanschen (314) verbunden ist, um zu gestatten, dass der Schlauch mit dem einzigen Lumen auf den mehreren Flanschen befestigt wird.

17. Fluidadapter nach Anspruch 2, wobei der Zwischenschlauch (348) durchsichtig und dazu konfiguriert ist, eine Sichtinspektion davon zu gestatten.

18. Fluidadapter nach Anspruch 1, wobei das Gehäuse aus einem Thermoplast hergestellt ist.

19. Fluidadapter nach Anspruch 1, wobei das Gehäuse aus einem Material hergestellt ist, das aus der aus einem phthalatfreien Polyvinylchlorid, einem HDPE-Kunststoff, einem PP-Kunststoff und irgendeiner Kombination davon bestehenden Gruppe ausgewählt ist.

20. Fluidadapter nach Anspruch 1, wobei mindestens eine Öffnung eine Presspassung aufweist und wobei eine andere Öffnung eine bezüglich der Presspassung im Verhältnis losere Passung aufweist.

21. Fluidadapter nach Anspruch 1, wobei der einzige Auslass (304) einen Halsteil mit mehreren Flanschen (314) aufweist, wobei die mehreren Flansche eine widerhakenartige Konfiguration zur abgedichteten Verbindung mit dem Schlauch bilden.

## Revendications

1. Adaptateur pour fluides (300) destiné à la connexion entre une soupape et un tube ayant une lumière unique, l'adaptateur pour fluides comprenant :
un boîtier (302) ayant une extrémité distale (304) et une extrémité proximale (306) ;
ladite extrémité proximale (306) ayant une première ouverture (326) se raccordant à une première lumière pratiquée à travers ledit boîtier ;
ladite extrémité proximale (306) ayant une deuxième ouverture (324) séparée par ledit premier orifice d'une certaine distance, ladite deuxième ouverture étant un orifice à extrémité fermée se raccordant à une deuxième lumière ; et
ladite extrémité distale ayant ladite première lumière raccordée à une sortie unique (304), ladite sortie se raccordant au tube ayant la lumière unique,
**caractérisé en ce que** ledit boîtier (302) comprend en outre une languette (320), ladite languette (320) étant raccordée de manière détachable sur un orifice secondaire (286) de la soupape (222) et ladite languette (320) étant configurée pour fermer l'orifice secondaire afin d'empêcher l'utilisateur de commuter l'orifice secondaire et ledit boîtier étant configuré pour être raccordé de manière détachable à la soupape.

2. Adaptateur pour fluides (300) selon la revendication 1, destiné à la connexion entre une soupape ayant une pluralité d'orifices et un tube, le tube ayant une lumière unique, ledit boîtier se raccordant à un tube intermédiaire (348) ; et ledit tube intermédiaire (348) étant raccordé au tube pour permettre au fluide de traverser le tube et la soupape.

3. Adaptateur pour fluides selon la revendication 2, dans lequel ledit tube intermédiaire est raccordé à un organe (304) ayant une extrémité distale avec une portion de col configurée pour permettre au tube ayant la lumière unique de s'attacher par-dessus ladite portion de col.

4. Adaptateur pour fluides selon la revendication 3, dans lequel ladite portion de col (304) dudit organe comprend une pluralité de brides (314).

5. Adaptateur pour fluides selon la revendication 4, dans lequel ladite pluralité de brides (314) est configurée pour former une configuration en forme de barbes pour retenir le tube sur celles-ci de manière étanche.

6. Adaptateur pour fluides selon la revendication 5, dans lequel ladite pluralité de brides (314) a au moins une bride avec un premier diamètre et au moins une autre bride avec un deuxième diamètre, ledit premier diamètre et ledit deuxième diamètre étant des diamètres de tailles différentes.

7. Adaptateur pour fluides selon la revendication 5, dans lequel ladite pluralité de brides (314) est dimensionnée de manière à faciliter la rétention du tube de manière étanche.

8. Adaptateur pour fluides selon la revendication 5, dans lequel ladite pluralité de brides (314) a au moins une bride avec un premier diamètre et au moins une autre bride avec un deuxième diamètre, ledit premier diamètre et ledit deuxième diamètre étant des diamètres de tailles différentes pour faciliter la rétention du tube.

9. Adaptateur pour fluides selon la revendication 2, dans lequel ledit boîtier est généralement cylindrique et dimensionné de manière à pouvoir être manipulé à la main.

10. Adaptateur pour fluides selon la revendication 9, dans lequel ledit tube intermédiaire (348) a une certaine longueur, ladite longueur étant appropriée pour faire tourner la soupape raccordée au boîtier sans désengager ledit boîtier de la soupape.

11. Adaptateur pour fluides selon la revendication 1, dans lequel ladite première lumière est généralement cylindrique.

12. Adaptateur pour fluides selon la revendication 1, dans lequel ladite deuxième lumière est généralement cylindrique et se termine à une extrémité.

13. Adaptateur pour fluides selon la revendication 1, dans lequel ledit boîtier est fabriqué en un matériau flexible.

14. Adaptateur pour fluides selon la revendication 1, dans lequel au moins l'une de ladite première lumière et de ladite deuxième lumière a une première taille qui est relativement plus petite qu'une autre de ladite première lumière et de ladite deuxième lumière afin de recevoir la pluralité d'orifices de la soupape.

15. Adaptateur pour fluides selon la revendication 14, dans lequel au moins une lumière se termine à une extrémité pour fermer au moins l'un de la pluralité des orifices de la soupape, ledit orifice fermé étant un orifice d'évent.

16. Adaptateur pour fluides selon la revendication 3, dans lequel ledit tube intermédiaire (348) est raccordé à un organe bridé ayant une pluralité de brides (314) pour permettre au tube ayant la lumière unique de s'attacher par-dessus la pluralité de brides.

17. Adaptateur pour fluides selon la revendication 2, dans lequel ledit tube intermédiaire (348) est transparent et est configuré de manière à permettre une inspection visuelle de celui-ci.

18. Adaptateur pour fluides selon la revendication 1, dans lequel ledit boîtier est fabriqué en un matériau thermoplastique.

19. Adaptateur pour fluides selon la revendication 1, dans lequel ledit boîtier est fabriqué en un matériau choisi parmi le groupe constitué de chlorure de polyvinyle exempt de phtalates, d'un plastique HDPE, d'un plastique PP, et d'une combinaison quelconque de ceux-ci.

20. Adaptateur pour fluides selon la revendication 1, dans lequel au moins une ouverture a un ajustement par compression, et dans lequel au moins une autre ouverture a un ajustement relativement plus lâche par rapport audit ajustement par compression.

21. Adaptateur pour fluides selon la revendication 1, dans lequel ladite sortie unique (304) a une portion de col avec une pluralité de brides (314), ladite pluralité de brides formant une configuration en forme de barbes pour se raccorder au tube de manière étanche.
